(19) Europäisches Patentamt / European Patent Office / Office européen des brevets

(11) **EP 4 390 944 A1**

(12) **EUROPEAN PATENT APPLICATION**

(43) Date of publication:
**26.06.2024 Bulletin 2024/26**

(21) Application number: **22214850.4**

(22) Date of filing: **20.12.2022**

(51) International Patent Classification (IPC):
**G16H 10/40** (2018.01)    **G16H 40/40** (2018.01)

(52) Cooperative Patent Classification (CPC):
**G16H 10/40; G16H 40/40**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Tecan Trading AG**
**8708 Männedorf (CH)**

(72) Inventors:
• **KELLER, Michael**
**8494 Bauma (CH)**
• **WALSER, Simon**
**7204 Trimmis (CH)**
• **BAUER, Christina**
**8005 Zurich (CH)**
• **SCHUSTER, Guido**
**8712 Stäfa (CH)**

(74) Representative: **Sykora & König Patentanwälte PartG mbB**
**Maximilianstraße 2**
**80539 München (DE)**

(54) **CLASSIFYING PIPETTING PROCEDURES WITH MEDIAN OF PRESSURE CURVES**

(57)    A method for classifying a specific pipetting procedure of a laboratory automation device (10) comprises: determining a plurality of pressure curves (40) of a plurality of pipetting procedures, which are equivalent to the specific pipetting procedure; determining a median curve (42) from the plurality of pressure curves (40), wherein for each time point of the median curve, a median value of the values of the plurality of pressure curves (40) at this time point is calculated; determining a median curve range (50) containing the median curve (42), the median curve range having an upper limit curve (44) and a lower limit curve (46) being distant a deviation threshold (52) from the median curve (42); controlling the laboratory automation device (10) to perform the specific pipetting procedure and measuring a specific pressure curve (54) during the specific pipetting procedure; determining a deviation value (56) of the specific pressure curve with respect to the median curve range, wherein the deviation value is indicative of the specific pressure curve (54) leaving the median range (50); and classifying the specific pipetting procedure as erroneous, when the deviation value (56) is greater than a threshold.

**Fig. 5**

EP 4 390 944 A1

## Description

FIELD OF THE INVENTION

The invention relates to a method, a computer program, a computer-readable medium and a control device for classifying a pipetting procedure of a laboratory automation device. Furthermore, the method relates to a laboratory automation device.

BACKGROUND OF THE INVENTION

Laboratory automation devices are used for automating tasks of a laboratory assistant, which, for example, tests a patient for specific diseases. Usually, a sample of the patient's blood, urine, stool, etc. is taken and analysed by means of a biochemical procedure. Such a procedure consists in various operations like adding substances, incubating, separating, etc. and a measurement process which quantitatively or qualitatively measures the amount or presence of a substance indicating the specific disease.

Usually, such an assay procedure comprises several pipetting procedures, in which a liquid is aspirated from a sample container and dispensed in a further container, for example, for adding substances. Every time it is important to know, whether the pipetting procedure was correctly executed, i.e. that the correct volume of liquid was aspirated or dispensed. Failures may occur due to the cloths of the pipette tip, bubbles in the liquid, short samples, etc.

It is known to record a pressure curve during a pipetting procedure and to classify the pipetting quality based on comparison to standard curves. For example, EP 1 412 759 B1 and US 6 938 504 B2 propose to determine an average curve of a set of statistically significant pressure-time curves, which were measured during a dosing process with identical equipment and working parameter set. A state variable setpoint range may be defined as following the average curve, in which case then, to evaluate a dosing process, it is determined whether the time characteristic of the at least one state variable is within the state variable setpoint range.

DESCRIPTION OF THE INVENTION

It is an objective of the invention to automate and simplify the finding of error during an assay procedure performed by a laboratory automation device. It is a further objective to improve the accurateness of the classification of pipetting procedures. It is a further objection of the invention to reduce false positives in classification of erroneous pipetting procedures.

This objective is achieved by the subject-matter of the independent claims. Further exemplary embodiments are evident from the dependent claims and the following description.

A first aspect of the invention relates to a method for classifying a specific pipetting procedure of a laboratory automation device. The method may be performed automatically by the laboratory automation device and/or a control device of the laboratory automation device. A control device of a laboratory automation device, which control device is adapted for performing the method, also is a further aspect of the invention.

According to an embodiment of the invention, the method comprises: determining a plurality of pressure curves of a plurality of pipetting procedures, which are equivalent to the specific pipetting procedure. As described below, the plurality of pressure curves may be measured or may be simulated. The measuring or simulation may be performed for a plurality of equivalent pipetting procedures, which have the same or nearly the same processing parameters. Having the same or nearly the same processing parameters, which may only deviate within a range from average and/or predefined processing parameters, may define equivalent pipetting procedures.

A pressure curve may be a set of pressure values over time. The pressure curve may be a curve over time.

According to an embodiment of the invention, the method further comprises: determining a median curve from the plurality of pressure curves, wherein for each time point of the median curve, a median value of the values of the plurality of pressure curves at this time point is calculated. The median value of a set of values may be any value such that at least half of the values is less than or equal to the median value and at least half is greater than or equal to the median value. Using the median of pressure curves to calculate a standard curve for the pipetting procedure is very robust even if some of the curves are error curves, for example even for more than 20% errors. The plurality of pressure curves comprises at least 10 pressure curves or at least more than 10 pressure curves, when the median curve and median curve range are determined.

The plurality of pressure curves may be timely aligned by finding a time point at which a pressure changes starts. This point may be calibrated to the beginning time point.

According to an embodiment of the invention, the method further comprises: determining a median curve range containing the median curve, the median curve range having an upper limit curve and a lower limit curve being distant a deviation threshold from the median curve. A range containing the median curve is defined, in which unproblematic pressure curves stay. This range may be defined by an upper range curve and a lower range curve. Both of this curves may be defined by adding a threshold to the median curve and subtracting a threshold to the median curve. This threshold may be fixed and may be predefined in the device performing the method.

According to an embodiment of the invention, the method further comprises: controlling the laboratory automation device to perform the specific pipetting procedure and measuring a specific pressure curve during the specific pipetting procedure. Whenever a pipetting procedure is performed, which is equivalent to the pipetting procedures from which the median curve and the median curve range have been computed, a pressure is measured during the pipetting and a pressure curve is generated from the measurements. Usually, a pressure sensor is present, which is connected to the pipette and/or to the pipette holder and which can be used for measuring the pressure curve of the pipetting procedure.

According to an embodiment of the invention, the method further comprises: determining a deviation value of the specific pressure curve with respect to the median curve range, wherein the deviation value is indicative of the specific pressure curve leaving the median range. It is determined, whether the specific pressure curves leaves the median pressure range and/or how much of the specific pressure is outside of the median pressure range. For example, the deviation value may solely indicate, whether the specific pressure curve leaves the median curve range at least at one time point. The deviation value also may be a measure of the percentage of time, when the specific pressure curve leaves the median curve range.

According to an embodiment of the invention, the method further comprises: classifying the specific pipetting procedure as erroneous, when the deviation value is greater than a threshold. A warning message and/or error message may be generated and/or output, when a pipetting procedure is performed, which has a deviation value higher than a threshold.

The classification may be seen as outlier detection, which classifies, which pressure curve is an outlier (i.e. erroneous pipetting procedure) or not.

It may be that, when the laboratory automation device performs a plurality of equivalent pipetting procedures, then for every pipetting procedure, the pressure curve and/or the deviation value is stored, and that, when all equivalent pipetting procedures are performed, a user of the laboratory automation device can check, which pipetting procedures have been successful and which one should be looked at. For example, the pressure curves of the pipetting procedure classified as erroneous may be displayed and the user may decide individually, whether really an error was present or not. Errors can be visually shown with the allowed range. The method can be used to postprocess recorded pressure curves and find unusual pressure curves very fast.

For example, the classification method may be automatically activated, whenever a large amount of equal samples (such as more than 10, 20 or 100) is processed and equivalent pipetting procedures are performed. In the beginning, some pipetting procedures may be performed without classification and pressure curves may be measured, which then are used for determining the median curve and the median curve range.

According to an embodiment of the invention, the deviation threshold is time dependent. The median curve range limits may not have the same distance to the median curve over time. For example, the deviation threshold at a time point may depend on a spread of the plurality of pressure curves at this time point. As an example, the standard deviation of the values of the plurality of pressure curves at this time point may be used as deviation threshold.

According to an embodiment of the invention, the deviation threshold at a time point depends on, or is proportional to, the median absolute deviation of the values of the plurality of pressure curves at that time point. The median absolute deviation may be defined as the median value of the absolute deviations from the median value. The absolute deviation may be the distance of the respective value to the median value at this time point. The deviation threshold again may be the median of these distances.

The expected deviation to the median curve may be calculated using the MAD (Median Absolute Deviation) algorithm. This may allow for a robust calculation of the expected deviation even if 20% of the curves are error curves.

According to an embodiment of the invention, the deviation value depends on an area of the specific pressure curve outside of the median range. The distances of the specific pressure curve to the upper and lower limit curve, when the specific pressure curve is outside of the median curve range, may be calculated and summed over and/or integrated, in the time regions, when the specific pressure curve is outside of the median curve range.

According to an embodiment of the invention, the deviation value depends on a duration of the specific pressure curve outside of the median range. It is also possible that the one or more time periods, when the specific pressure curve is outside of the median curve range, are determined and their duration is used as deviation value.

It may be that the classification comprises more than two outcomes. The deviation values may be classified as unproblematic, potentially problematic and erroneous.

According to an embodiment of the invention, the specific pipetting procedure is classified as unproblematic, when the deviation value is less than a first threshold. The first threshold may be greater than 0, i.e. it may be that, when the specific pressure curve is slightly outside of the median curve range, this also may be classified as unproblematic. The first threshold may be a warn threshold.

According to an embodiment of the invention, the specific pipetting procedure is classified as potentially problematic, when the deviation value is greater than the first threshold and less than a second threshold. In this case, the pressure curve may be provided with a warning message. The second threshold may be an error threshold.

It may be that the specific pipetting procedure is solely classified with respect to one threshold, which may be the second threshold, In this case, the specific pipetting procedure may be classified as unproblematic or erroneous.

According to an embodiment of the invention, the specific pipetting procedure is classified as erroneous, when the deviation value is greater than the second threshold. In this case, the pressure curve may be provided with an error message.

The warning messages and/or error messages may be output on an interface device to a user of the laboratory automation device.

According to an embodiment of the invention, the plurality of pressure curves is determined by controlling the laboratory automation device to perform the plurality of pipetting procedures and a pressure curve is measured during each pipetting procedure. For calibration, the laboratory automation device may perform some equivalent pipetting procedures, for example with a test liquid having known processing parameters, to collect the plurality of pressure curves. It also is possible that the plurality of pipetting procedures is performed with different samples and/or with samples to be processed by the laboratory automation device. This may be done in the beginning of a large assay procedure, in which a large amount of samples is pipetted. The calculation of the median curve and the following classification of further pressure curves may be automatically activated after a set number of pipetting procedures without further configu ration.

According to an embodiment of the invention, the plurality of pressure curves is determined by simulating the plurality of pipetting procedures. Alternatively or additionally, a plurality of pressure curves may be simulated with a model of the laboratory automation device, the pipette and the liquid.

During the simulation, the relevant parts of the laboratory automation device may be simulated during aspiration and/or dispensing. The relevant parts of the laboratory automation device and/or the model of the laboratory automation device used in the simulation may be the pipette, together with its orifice and/or the connected pressure system, such as hoses and pipes and the pump. Also, the liquid and the gas (usually air) inside these components may be simulated.

The model used in the simulation may comprise differential equations, which are solved during the simulation. The differentiable equation may model the properties of components over time. For example, the volume of the liquid and of the gas inside the components may be modelled as linear interconnected, oscillating masses.

In every simulation of one of the pressure curves, initial parameters may be varied, such that different pressure curves, having different pressure values at the same time point, are generated. The initial parameters may include geometric properties and the variations may comprise geometric tolerances. The initial parameters may comprise physical parameters, such as a filter resistance, environmental temperature, and the variations may comprise uncertainties in these physical properties. Also physical properties of the liquid, such as density, viscosity, etc., may be varied. The initial parameters also may comprise processing parameters of the laboratory automation device, such as a volume rate of the pump.

In this case, the classification of the pipetting quality is based on comparison to curves simulated by a model of the laboratory automation device. The usage of a simulation model allows to predict the pressure curves with high accuracy and without the need for performing real measurements in advance.

According to an embodiment of the invention, initial parameters of the simulation are selected by a Monte Carlo procedure, in which the parameters are varied in a range, for example around a theoretical, reference or default value. In Monte Carlo simulations, variations of the initial parameters are generated by using random numbers. This allows to predict the probability of different outcomes and therefore the expected deviations. A Monte Carlo method may pick combinations of such variations using random numbers. For example, the orifice radius for is provided as 0.8±0.005 mm, so a random value may be 0.803 mm. A liquid such as blood serum may have a viscosity of 1.9 mPa*s instead of the theoretical value 2.1 mPa*s and a range of the viscosity may be set to 2.1±0.2 mPa*s.

According to an embodiment of the invention, the initial parameters comprise physical parameters and/or processing parameters of the pipette, of the liquid and/or of the laboratory automation device. Parameters of the pipette may be a geometric shape, such as an orifice radius, an open angle of a cone ending in the orifice, and/or a filter resistance of a filter in the pipette. Parameters of the liquid may be a density, a viscosity, a surface tension and/or a wetting angle. Parameters of the laboratory automation device may be a movement speed of pipette tip, a flow rate of pump and/or a volume rate of pump.

According to an embodiment of the invention, the method further comprises: including the specific pressure curve into the plurality of pressure curves and updating the median curve and the median curve range. The median curve and the median curve range may be improved on the fly, i.e. with pressure curves that are classified based on already present pressure curves. It may be that solely a specific pressure curve, which is classified as unproblematic, is included into the plurality of pressure curves, i.e. that erroneous or problematic pressure curves are discarded for determining the median curve and the median curve range.

It is possible that measured pressure curves are added to a set of simulated pressured curves in this way.

The improvement of the median curve and the median curve range and/or the inclusion of the specific pressure curve into the plurality of pressure curves may be stopped after a specific number of pressure curves in the plurality of pressure curves, such as more than 100 and/or more than 500.

According to an embodiment of the invention, the pipetting procedure comprises aspirating and/or dispensing a liquid. It has to be noted that the method may be performed for aspiration and dispensing procedures.

According to an embodiment of the invention, a pipetting procedure is equivalent to the specific pipetting procedure, when at least one of: an equally formed pipette is used, the same liquid is aspirated and/or dispensed, the same volume of the liquid is to be dispensed and/or the same volume rate provided by a pump of the laboratory automation device is generated. These equal properties may result in standard values for specific parameters of the pipetting procedure, such as the initial parameters mentioned above. Deviations from these values may occur due to manufacturing tolerances (such as for the pipette) and/or tolerances of environment conditions (such as environment pressure and/or environmental temperature) and/or deviations of properties of the liquid (such as density, viscosity, etc.).

According to an embodiment of the invention, a pipetting procedure is equivalent to the specific pipetting procedure, when the laboratory automation device is controlled in the same way, for example for aspirating and/or dispensing the liquid. Equivalent pipetting procedures may have the same nominal flow rates and/or volume rates.

A further aspect of the invention relates to a computer program for classifying a specific pipetting procedure of a laboratory automation device, which computer program, when being executed by a processor, is adapted to carry out the steps of the method as described above and below. The computer program may be executed in a computing device, such as a control device of the laboratory automation device and/or a PC, which may be communicatively interconnected with the laboratory automation device. It also is possible that the method is performed by an embedded microcontroller of the laboratory automation device.

A further aspect of the invention relates to a computer-readable medium, in which such a computer program is stored. A computer-readable medium may be a floppy disk, a hard disk, an USB (Universal Serial Bus) storage device, a RAM (Random Access Memory), a ROM (Read Only Memory), an EPROM (Erasable Programmable Read Only Memory) or a FLASH memory. A computer-readable medium may also be a data communication network, e.g. the Internet and/or a cloud storage, which allows downloading a program code. In general, the computer-readable medium may be a non-transitory or transitory medium.

A further aspect of the invention relates to a laboratory automation device.

According to an embodiment of the invention, the laboratory automation device comprises a pipetting arm for carrying a pipette; a pump for changing a pressure in a volume connected to the pipette for aspirating and dispensing a liquid in the pipette; a pressure sensor for pressure measurements in the volume connected to the pipette and a control device for controlling the pump and the pipetting arm and for receiving a pressure signal from the pressure sensor, wherein the control device is adapted for performing the method as described above and below.

It has to be understood that features of the method as described in the above and in the following may be features of the control device, the computer program and the computer-readable medium as described in the above and in the following and vice versa.

These and other aspects of the invention will be apparent from and elucidated with reference to the embodiments described hereinafter.

BRIEF DESCRIPTION OF THE DRAWINGS

Below, embodiments of the present invention are described in more detail with reference to the attached drawings.

Fig. 1 schematically shows a laboratory automation device according to an embodiment of the invention.

Fig. 2 shows a flow diagram illustrating a method for classifying a pipetting procedure according to an embodiment of the invention.

Fig. 3 shows a diagram with pressure curves used in the method of Fig. 2.

Fig. 4 to 6 show diagrams with a pressure curve and median curve ranges used in the method of Fig. 2.

The reference symbols used in the drawings, and their meanings, are listed in summary form in the list of reference symbols. In principle, identical parts are provided with the same reference symbols in the figures.

DETAILED DESCRIPTION OF EXEMPLARY EMBODIMENTS

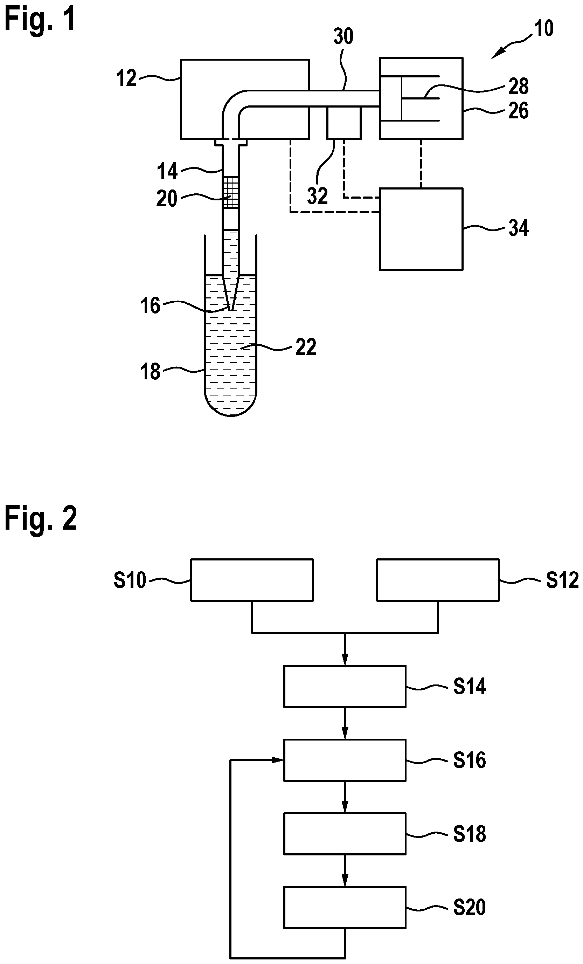

Fig. 1 schematically shows a laboratory automation device 10, which comprises an automatically movable pipetting arm 12 to which a pipette 14 is attached. As shown in Fig. 1, the pipette tip 16 of the pipette 14 may be lowered into a sample container 18 via the movable pipetting arm 12.

The pipetting arm 12 may move the pipette 14 and the pipette tip 16 in three dimensions, may lower the pipette tip 16 into the sample container 18 and may retract the pipette tip 16 therefrom.

The pipette 14 may contain a filter 20, which prevents entering substances from the pipette 14 into the remaining parts of the laboratory automation device 10.

In the sample container 18, a liquid 22 is contained. When the pipette tip 16 is immersed in the liquid in the sample container 18, the liquid can be aspirated into the pipette 14.

The laboratory automation device 10 comprises a pump 26, which is connected via a hose 30 with the pipette 14. With the pump 26, a pressure may be applied to the hose 30 and to the pipette 14, or more general to a volume 30 connected with the pipette 14, which causes the pipette 14 to aspirate or dispense the liquid 22 or air. For example, the pump 26 comprises a plunger 28, which is moved for generating underpressure and overpressure in the hose 30 and the pipette 14. Any other kind of pumps, e.g. peristaltic pumps, or in general any source of vacuum and pressure may be used. The source of vacuum and pressure may be controlled via valves.

A pressure sensor 32, which may be attached to the hose 30 and/or the pipette 14, is adapted for measuring a pressure in the hose 30 and/or the pipette 14.

A control device 34 of the laboratory automation device 10, which may be a part of the laboratory automation device 10 or connected thereto, may control the pipetting arm 12, the pump 26 and may receive a pressure signal from the pressure sensor 32.

Fig. 2 shows a flow diagram for a method for classifying a pipetting procedure of a laboratory automation device 10. The method may be performed by the control device 34, which may be a PC in data communication with the laboratory automation device 10 and/or a controller included the laboratory automation device 10.

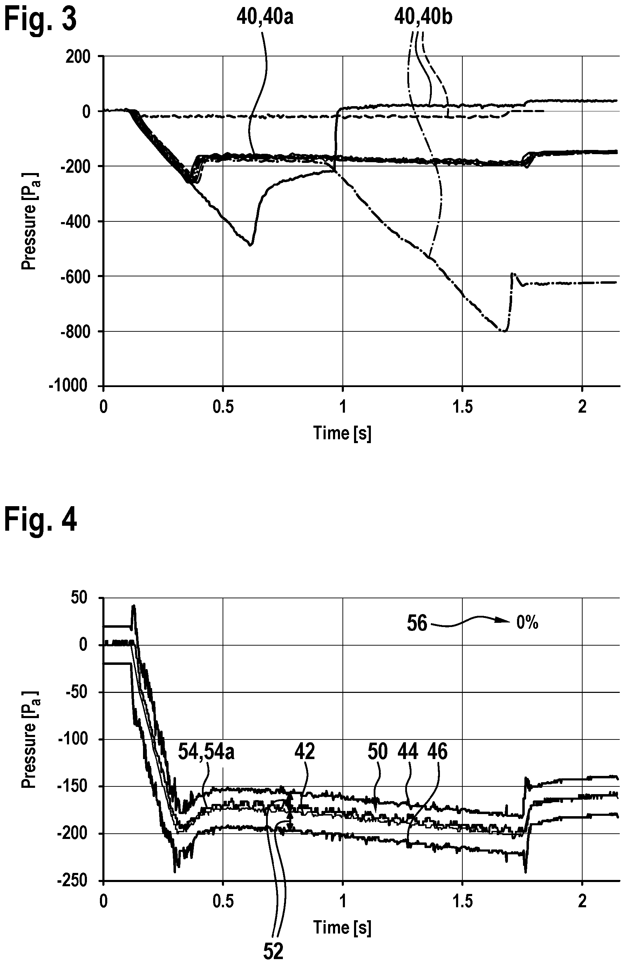

In step S10 and S12, a plurality of pressure curves 40 (see Fig. 3) of a plurality of pipetting procedures are determined. The curves 40 may be measured and/or simulated. In step S10, the plurality of pressure curves 40 is determined by measurements. In step S12, the plurality of pressure curves 40 is determined by simulation.

The pipetting procedures may relate to aspirating and/or dispensing the liquid 22. To this end, an underpressure (aspirating) or overpressure (dispensing) is generated in the pipette 14, such that liquid 22 is sucked into or blown out of the pipette 14. This procedure also may be simulated.

In any case, the curves 40 are determined with real and/or simulated pipetting procedures, which are equivalent to the specific pipetting procedure, which is classified later. A pipetting procedure may be equivalent to the specific pipetting procedure, when an equally formed pipette 14 is used, the same liquid 22 is aspirated and/or dispensed, the same volume of the liquid 22 is to be dispensed and/or the same volume rate provided by a pump 26 of the laboratory automation device 10 is generated.

It also may be that pipette procedures are differentiated by the laboratory automation device 10 with so called liquid classes, i.e. data structures in which parameters for the pipetting procedure are stored. These parameters may be parameters of the pipette, the liquid and/or the laboratory automation device 10. Pipetting procedure performed with the same liquid class may be seen as equivalent.

Also, a pipetting procedure may be equivalent to the specific pipetting procedure, when the laboratory automation device 10 is controlled in the same way. For example, there may be a specific program for controlling the pipetting procedure, which also uses the same parameters for all the pipetting procedures.

Steps S10 and S12 may be performed alternatively or in combination.

In step S10, the plurality of pressure curves 40 is determined by controlling the laboratory automation device 10 to perform the plurality of pipetting procedures and a pressure curve 40 is measured during each pipetting procedure.

Such pressure curves 40 are shown in Fig. 3, which are pressure curves 40 measured during aspiration of a liquid 22. As can be seen, most of the pressures curves 40a are rather similar, while some outlier curves 40b are quite different. However, also outlier curves 40b may be included into the plurality of pressure curves 40, since the median determination below is rather robust with respect to a small amount of outliers.

The measuring of pressure curves 40 in step S10 may be part of a larger assay procedure, in which a large amount of equivalent pipetting procedures is performed. As an example, it may be that 500 blood samples have to be aspirated and dispensed in wells. For example, the first 10 or 20 blood samples may be aspirated and dispensed and corresponding pressure curves 40 may be measured.

It may be that the aspiration procedures and the dispensing procedures are considered independently and that pressure curves 40 for the aspiration procedures are determined (which are considered as equivalent pipetting procedures), and that pressure curves 40 for the dispensing procedures are determined (which are considered as a further set of equivalent pipetting procedures).

In step S12, as a further option, the plurality of pressure curves 40a is determined by simulating the plurality of pipetting procedures. The simulation may be based on a model of the pipette, the liquid and the relevant parts of the laboratory automation device 10. The parameters of the simulation may be the parameters and/or properties mentioned above, such as geometric parameters of the pipette 14, the physical properties of the liquid 22 and processing parameters of the laboratory automation device 10.

As a rule, during simulation, no outlier curves 40b are produced, which may result in an even more accurate median calculation below.

To produce different pressure curves 40a by different simulation passes, initial parameters of the simulation are selected by a Monte Carlo procedure, in which the initial parameters are varied in a range around a theoretical, reference or default value. For example, for an opening radius of the orifice of the pipette 14, the desired opening radius, such as specified for the type of pipette 14, is used and the range may be based on manufacturing tolerances. In general, the initial parameters may comprise physical parameters of the pipette 14, of the liquid 22 and/or of the laboratory automation device 10.

In step S14, a median curve 42 and a median range 50, such as shown in Fig. 4 to 6, are determined. Step S14 may be performed, when the plurality of pressure curves 40 comprises at least 10, 20 or more pressure curves. The number of pressure curves here may be set as a parameter of the method.

The median curve 42 is determined from the plurality of pressure curves 40, such that for each time point of the median curve 42, a median value of the values of the plurality of pressure curves 40 at this time point is calculated.

A pressure curve 40 may be composed of a plurality of pressure values $p_i$ at time points ti (for example every 0.002s). At each time point ti for every pressure curve k of n pressure curves the pressure value $p_k$ is taken. The resulting n pressure values $p_1$ to $p_n$ for the time point ti are then ordered from smallest to greatest and the pressure median value $p_{Median}$ is selected such that half of the pressure values are lower than $p_{Median}$ and half of them are higher as follows:

If n is odd the median at time ti is $p_{Median} = median(p_k) = p_{(n+1)/2}$.

If n is even the median at time $t_i$ is $p_{Median} = median(p_k) = (p_{n/2} + p_{n/2+1})/2$.

The median curve 42 at time time ti has the median value $p_{Median}$ for the pressure values $p_1$ to $p_n$ for the time point ti as function value.

From the median curve 42, an upper limit curve 44 and a lower limit curve 46 are determined. This may be done by shifting the median curve 42 up and down with a fixed deviation threshold 52. Such a fixed deviation threshold 52 may be set as a parameter of the method. The upper limit curve 44 and the lower limit curve 46 have a distance to the median curve 42 by the deviation threshold 52.

The upper limit curve 44 and the lower limit curve 46 define a median curve range 50 between them, which is later used for classifying pressure curves 54.

It also may be that the deviation threshold 52 is determined from the plurality of pressure curves and/or that the deviation threshold 52 is time dependent.

In particular, the deviation threshold 52 at a time point may depend on a spread of the plurality of pressure curves 40 at this time point. As can be seen in Fig. 4, the median curve range 50 is wider in the interval between 1.7s and 1.8s compared to the time periods before and after, since there the pressure curves 40 differ stronger from each other. Any measure quantifying the local and/or global spread of the pressure curves 40 may be used.

As one example, the deviation threshold 52 at a time point ti may depend on the median absolute deviation of the values of the plurality of pressure curves 40 at that time point $t_i$.

The Median Absolute Deviation (MAD) is calculated like the median but using the absolute value of the difference of $p_k$ to $p_{Median}$ instead of $p_k$.

$$MAD = median(abs(p_k\text{-}p_{Median})).$$

The deviation threshold 52 may be the median absolute deviation multiplied with a factor, which may be preselected.

In step S16, a pressure curve 54 is measured and compared with the median curve range 50.

The laboratory automation device 10 is controlled to perform a specific pipetting procedure, which is equivalent to the ones of the pressure curves 40, and a specific pressure curve 54 is measured during the specific pipetting procedure. The pressure curves 40 may be seen as calibrating pressure curves and the pressure curve 54 as curve to be classified.

After that, a deviation value 56 of the specific pressure curve 54 with respect to the median curve range 50 is determined. The deviation value 56 is indicative of the specific pressure curve 54 leaving the median range 50.

In the example of Fig. 4, the pressure curve 54 is completely within the median curve range 50 and the deviation value 56 is 0%.

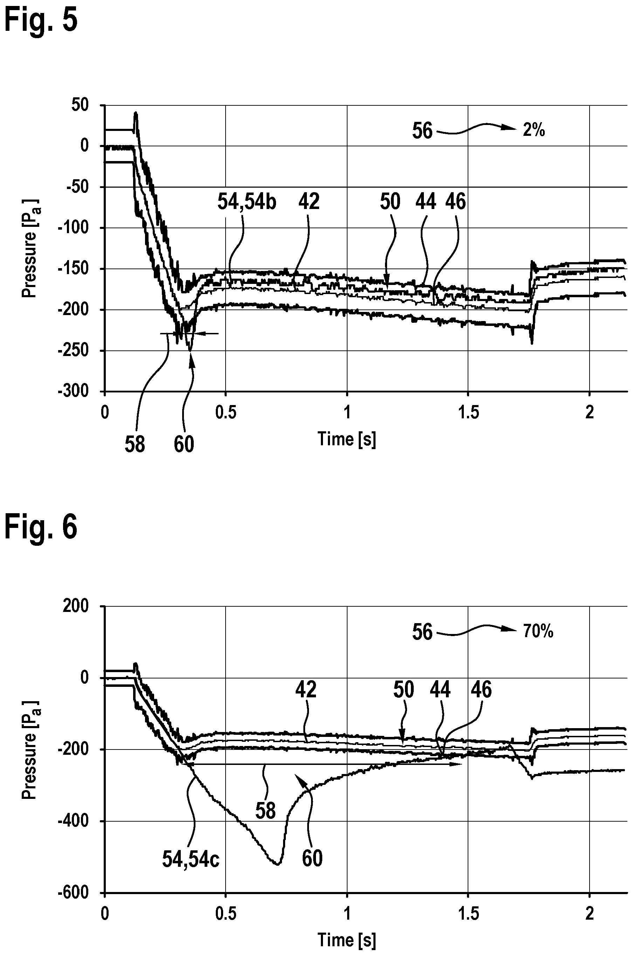

In the example of Fig. 5, the pressure curve 54 is outside of the median curve range 50 in a short time interval and the deviation value 56 is different from 0.

Here, the deviation value 56 depends on a duration 58 of the specific pressure curve outside of the median curve range 50, which in the present case is 2% of the duration of the pipetting procedure. The start time and end time of pipetting procedure may be set to fixed values, such as 0s and 2s, which may be parameters of the method, which can be set in the beginning and/or which may be determined from the plurality of pressure curves 40.

As a further example, the deviation value 56 may depend on an area 60 of the specific pressure curve outside of the median range 50. In this case, the area 60 outside of the median curve range 50, between the pressure curve 54 and the upper limit curve 44 and the lower limit curve 46, respectively, can be determined, for example, by discrete integration.

In step S18, the specific pipetting procedure is classified dependent on the deviation value 56.

The specific pipetting procedure may be classified as erroneous and/or problematic, when the deviation value is greater than a threshold.

For example, pressure curves 54 and/or the corresponding pipetting procedure may be classified into three categories, unproblematic, problematic and erroneous.

The specific pipetting procedure may be classified as unproblematic, when the deviation value 56 is less than a first threshold which need not be 0. For example, the pressure curves 54a and 54b may be classified as unproblematic.

The specific pipetting procedure is classified as problematic, when the deviation value 56 is greater than the first threshold and less than a second threshold.

The specific pipetting procedure may be classified as erroneous, when the deviation value 56 is greater than the second threshold. This is the case for the pressure curve 54c shown in Fig. 6. Here, the pressure curve 54c is outside of the median curve range for about 70% of the duration of the pipetting procedure, which indicates an error.

The pressure curve 40 may be stored in the control device 34 and may be displayed together with the median curve 42 and/or the median curve range 50 on a display. A user of the laboratory automation device 10 may view the curves and may decide, whether the classification was correct or not. This may be done in particular for the problematic and/or erroneous curves.

When the pressure curves 54 and/or the corresponding pipetting procedure is classified as not unproblematic, such as problematic or erroneous, a warning message and/or error message may be triggered, which also may be displayed on a display.

It also may be that the pressure curves 40, that have been measured in step S10, are also classified such as described with respect to steps S16 and S18, after that the median curve 42 and the median curve range 50 have been determined with respect to them. In such a way, all measured curves 40 may be classified and optionally inspected by a user.

In optional step S20, median curve range 50 is updated based on the specific pressure curve 54. The specific pressure curve 54 may be included into the plurality of pressure curves 40 and the median curve 42 and the median curve range 50 may be recalculated with respect to the enlarged set of pressure curves 40.

It may be that solely a specific pressure curve 54a, which is classified as unproblematic, is included into the plurality of pressure curves 40. It also may be that solely a fixed number of pressure curves 54 is included into the plurality of pressure curves 40, such as the first 100 measured pressure curves 54. After that, step S20 may be skipped.

Another option may be to retain the last, such as the last 100, measured pressure curves 54 to continually update the median absolute deviation range. I.e. solely a number of the last measured curves 54 is used as plurality of pressure curves 40.

The method continues in step S16, where the next pipetting procedure is performed. This may be done until all samples of an assay procedure have been processed.

The median curve 42, the median curve range 50 and/or the parameters set for the classification, such as thresholds, may be saved in a liquid class for the pipetting procedure and may be used again, when the same liquid class is used for a pipetting procedure.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. A single processor or control device or other unit, such as an FPGA, may fulfil the functions of several items recited in the claims. The mere fact that certain measures are recited in mutually different dependent claims does not indicate that a combination of these measures cannot be used to advantage. Any reference signs in the claims should not be construed as limiting the scope.

LIST OF REFERENCE SYMBOLS AND NOMENCLATURE

| | |
|---|---|
| 10 | laboratory automation device |
| 12 | pipetting arm |
| 14 | pipette |
| 16 | pipette tip |
| 18 | sample container |
| 20 | filter |
| 22 | liquid |
| 26 | pump |
| 28 | plunger |
| 30 | hose / volume |
| 32 | pressure sensor |
| 34 | control device |
| 40 | pressure curves |
| 40a | unproblematic curves |
| 40b | outlier curves |
| 42 | median curve |
| 44 | upper limit curve |
| 46 | lower limit curve |
| 50 | median curve range |
| 52 | deviation threshold |
| 54 | measured pressure curve |
| 54a | unproblematic curve |
| 54b | problematic curve |
| 54c | erroneous curve |
| 56 | deviation value |

**Claims**

**1.** A method for classifying a specific pipetting procedure of a laboratory automation device (10), the method comprising:

determining a plurality of pressure curves (40) of a plurality of pipetting procedures, which are equivalent to the specific pipetting procedure;

determining a median curve (42) from the plurality of pressure curves (40), wherein for each time point of the median curve, a median value of the values of the plurality of pressure curves (40) at this time point is calculated;

determining a median curve range (50) containing the median curve (42), the median curve range having an upper limit curve (44) and a lower limit curve (46) being distant a deviation threshold (52) from the median curve (42);

controlling the laboratory automation device (10) to perform the specific pipetting procedure and measuring a specific pressure curve (54) during the specific pipetting procedure;

determining a deviation value (56) of the specific pressure curve with respect to the median curve range, wherein the deviation value is indicative of the specific pressure curve (54) leaving the median range (50);

classifying the specific pipetting procedure as erroneous, when the deviation value (56) is greater than a threshold.

2. The method of claim 1,

wherein the deviation threshold (52) is time dependent; and/or

wherein the deviation threshold (52) at a time point depends on a spread of the plurality of pressure curves (40) at this time point.

3. The method of claim 1 or 2,
wherein the deviation threshold (52) at a time point depends on the median absolute deviation of the values of the plurality of pressure curves (40) at that time point.

4. The method of one of the previous claims,

wherein the deviation value (56) depends on an area (60) of the specific pressure curve outside of the median range (50); and/or

wherein the deviation value (56) depends on a duration (58) of the specific pressure curve outside of the median range (50).

5. The method of one of the previous claims,

wherein the specific pipetting procedure is classified as unproblematic, when the deviation value (56) is less than a first threshold;

wherein the specific pipetting procedure is classified as potentially problematic, when the deviation value (56) is greater than the first threshold and less than a second threshold;

wherein the specific pipetting procedure is classified as erroneous, when the deviation value (56) is greater than the second threshold.

6. The method of one of the previous claims, wherein the plurality of pressure curves (40) is determined by controlling the laboratory automation device (10) to perform the plurality of pipetting procedures and a pressure curve (40) is measured during each pipetting procedure.

7. The method of one of the previous claims, wherein the plurality of pressure curves (40) is determined by simulating the plurality of pipetting procedures.

8. The method of one of the previous claims,

wherein initial parameters of the simulation are selected by a Monte Carlo procedure, in which the parameters are varied in a range;

wherein the initial parameters comprise physical parameters of the pipette (14), of the liquid (22) and/or of the laboratory automation device (10).

9. The method of one of the previous claims, further comprising:

including the specific pressure curve (54) into the plurality of pressure curves (40) and updating the median curve (42) and the median curve range (50); and/or

wherein solely a specific pressure curve (54a), which is classified as unproblematic, is included into the plurality of pressure curves (40).

10. The method of one of the previous claims, wherein the plurality of pressure curves (40) comprises at least 10 pressure curves, when the median curve (42) and the median curve range (50) are determined.

11. The method of one of the previous claims,

wherein the pipetting procedure comprises aspirating and/or dispensing a liquid (22); and/or

wherein a pipetting procedure is equivalent to the specific pipetting procedure, when at least one of: an equally formed pipette (14) is used, the same liquid (22) is aspirated and/or dispensed, the same volume of the liquid (22) is to be dispensed and/or the same volume rate provided by a pump (26) of the laboratory automation device (10) is generated; and/or

wherein a pipetting procedure is equivalent to the specific pipetting procedure, when the laboratory automation device (10) is controlled in the same way.

12. A computer program for classifying a specific pipetting procedure of a laboratory automation device (10), which computer program, when being executed by a processor, is adapted to carry out the steps of the method of one of the previous claims.

13. A computer-readable medium, in which a computer program according to claim 13 is stored.

14. A control device (34) of a laboratory automation device (10) adapted for performing the method of one of claims 1 to 12.

15. A laboratory automation device (10), comprising:

a pipetting arm (12) for carrying a pipette (14);
a pump (26) for changing a pressure in a volume (30) connected to the pipette (14);
a pressure sensor (32) for pressure measurements in the volume (30) connected to the pipette (14);
a control device (34) for controlling the pump (26) and the pipetting arm (12) and for receiving a pressure signal from the pressure sensor (32);
wherein the control device (34) is adapted for performing the method of one of claims 1 to 12.

**Fig. 1**

**Fig. 2**

# Fig. 3

# Fig. 4

## Fig. 5

## Fig. 6

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/070049 A1 (ZIEGLER GUENTER [DE] ET AL) 12 March 2009 (2009-03-12) | 1-14 | INV. G16H10/40 G16H40/40 |
| Y | * paragraphs 12, 17, 60, 24, 41, 30, 31, 18, 32, 22, 47, 26, 27; figures 1-7 * | 15 | |
| Y | US 2022/026455 A1 (KELLER MICHAEL [CH]) 27 January 2022 (2022-01-27) * paragraphs 61-65 * | 15 | |
| A | US 2007/025882 A1 (ZUPPIGER ADI [CH] ET AL) 1 February 2007 (2007-02-01) * paragraphs 17-25, 51-60 * | 7,8 | |

| TECHNICAL FIELDS SEARCHED (IPC) |
|---|
| G16H |

The present search report has been drawn up for all claims

| Place of search | Date of completion of the search | Examiner |
|---|---|---|
| Munich | 7 June 2023 | Wittke, Claudia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 21 4850

This annex lists the patent family members relating to the patent documents cited in the above-mentioned European search report.
The members are as contained in the European Patent Office EDP file on
The European Patent Office is in no way liable for these particulars which are merely given for the purpose of information.

07-06-2023

| Patent document cited in search report | | Publication date | Patent family member(s) | | Publication date |
|---|---|---|---|---|---|
| US 2009070049 | A1 | 12-03-2009 | EP | 2031403 A1 | 04-03-2009 |
| | | | US | 2009070049 A1 | 12-03-2009 |
| | | | WO | 2009027071 A1 | 05-03-2009 |
| US 2022026455 | A1 | 27-01-2022 | CN | 113272655 A | 17-08-2021 |
| | | | EP | 3671221 A1 | 24-06-2020 |
| | | | EP | 4030170 A1 | 20-07-2022 |
| | | | JP | 2022515119 A | 17-02-2022 |
| | | | US | 2022026455 A1 | 27-01-2022 |
| | | | WO | 2020126694 A1 | 25-06-2020 |
| US 2007025882 | A1 | 01-02-2007 | DE | 202006010293 U1 | 31-08-2006 |
| | | | US | 2007025882 A1 | 01-02-2007 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1412759 B1

- US 6938504 B2